# EUROPÄISCHE PATENTSCHRIFT

(11) **EP 0 576 393 B1**
(45) Veröffentlichungstag und Bekanntmachung des Hinweises auf die Patenterteilung: **10.04.1996**
(21) Anmeldenummer: 93810387.6
(22) Anmeldetag: 27.05.1993
(51) Int. Cl.: C08G 59/68, C08G 18/20, C07D 521/00

(54) **Härtbare Zusammensetzungen auf Basis von Epoxidharzen oder Gemischen von Epoxidharzen und Polyisocyanaten enthaltend Trisimidazolyltriazine**
Curable compositions based on epoxy resins or mixtures of epoxy resins and polyisocyanates containing a trisimidazolyltriazone
Compositions durcissables à base de résines époxydes ou des mélanges de résines époxydes et des polyisocyanates contenant un trisimidazolyltriazine

(30) Priorität: 05.06.1992 CH 1810/92
(43) Veröffentlichungstag der Anmeldung: 29.12.1993
(73) Patentinhaber: CIBA-GEIGY AG, CH-4002 Basel (CH)
(72) Erfinder: Mühlebach, Andreas, Dr., CH-1782 Belfaux (CH); Flury, Peter, Dr., CH-4204 Himmelried (CH)

(56) Entgegenhaltungen:
- EP-A- 0 050 939
- EP-A- 0 114 784
- DE-A- 1 720 526
- US-A- 3 308 122
- CHEMICAL ABSTRACTS, vol. 91, no. 16, 15. Oktober 1979, Columbus, Ohio, US; abstract no. 125037c, 'Resin compositions for delustering powdered coating compositions' Seite 81 ; & JP-A-79 040 831
- DATABASE WPI Derwent Publications Ltd., London, GB; AN 78-34424A & JP-A-53 034 896

## Beschreibung

Die Erfindung betrifft härtbare Zusammensetzungen auf Basis von Epoxidharzen oder Gemischen von Epoxidharzen und Polyisocyanaten, ein Verfahren zur Härtung der genannten Harze oder Harzgemische und die mit Hilfe der Zusammensetzungen erhältlichen Produkte.

Für Zusammensetzungen der eingangs genannten Art sind verschiedene Verbindungen als Härter und/oder Härtungsbeschleuniger gebräuchlich, beispielsweise Dicyandiamid, Chlortoluron^{®} oder Imidazol und Imidazolderivate.

Auch substituierte Triazine sind bereits für diese Zwecke bekannt. So beschreiben die Japanische Offenlegungsschrift 60/84,283 (1985) die Verwendung von 2,4-Diamino-6- [2-(2-phenyl-1H-imidazo1-1-yl)ethyl]triazin als Härter für Polyepoxidharze und die Japanische Offenlegungsschrift 59/196,377 (1984) härtbare Zusammensetzungen auf Basis von Epoxidharzen, Butadiencopolymeren und Bisphenol, die ausserdem noch 2,4-Diamino-6-[2-(2-methyl-1H-imidazol-1-yl)ethyl]triazin und Dicyandiamid enthalten, und deren Verwendung als Strukturklebstoff.

Alle genannten Zusammensetzungen können jedoch nicht voll befriedigen. So sind sie entweder bei niedrigen Temperaturen, wie z. B. bei 100 - 110 °C, zu wenig reaktiv oder aber ausreichend reaktiv, jedoch nicht lagerstabil genug. Bei vielen Zusammensetzungen des Standes der Technik lassen weiterhin andere wichtige Eigenschaften, wie die Härtungsgeschwindigkeit, d. h. der in einem bestimmten Zeitraum und bei bestimmter Temperatur erreichbare Härtungsgrad, und insbesondere die Klebe- und Haftfähigkeit des gehärteten Materials zu wünschen übrig.

Es ist die Aufgabe der vorliegenden Anmeldung, eine härtbare Zusammensetzung auf Basis von Epoxidharzen oder Gemischen von Epoxidharzen und Polyisocyanaten zur Verfügung zu stellen, die gute Reaktivität, auch schon bei einer Temperatur von 100 - 110 °C, und trotzdem gute Lagerstabilität zeigt.

Zur Lösung der genannten Aufgabe werden gemäss der vorliegenden Erfindung härtbare Zusammensetzungen auf Basis von Epoxidharzen oder Gemischen von Epoxidharzen und Polyisocyanaten vorgeschlagen, die mindestens eine Verbindung der Formel 1 enthalten: 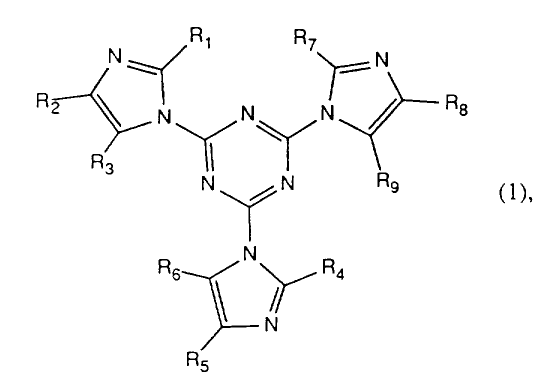 worin R₁, R₂, R₃, R₄, R₅, R₆, R₇, R₈ und R₉ jeweils unabhängig voneinander Wasserstoff, Alkyl, Aryl, Arylalkyl, oder Halogen bedeuten und R₂ zusammen mit R₃ sowie R₅ zusammen mit R₆ und R₈ zusammen mit R₉ ausserdem auch jeweils einen an dieser Stelle annellierten Benzolring darstellen können.

Es hat sich nämlich gezeigt, dass die Verwendung von Verbindungen der oben genannten Formel 1 als Härter oder Co-Härter bzw. Härtungsbeschleuniger in Zusammensetzungen auf Basis von Epoxidharzen oder Gemischen von Epoxidharzen und Polyisocyanaten zu guter Reaktivität, besonders bei niedrigen Temperaturen, bei gleichzeitig ausreichender Lagerbeständigkeit der Zusammensetzungen führt. Die erfindungsgemässen Zusammensetzungen weisen also ein besonders gutes Stabilitäts-/Reaktivitätsverhältnis auf, obwohl diese beiden Eigenschaften normalerweise in Widerspruch zueinander stehen. Um nämlich eine lange Lagerstabilität zu erreichen, ist es günstig, ein möglichst wenig reaktives Vernetzungs- oder Härtungsmittel zu verwenden. Wenig reaktive Vernetzungsmittel, führen aber zu langen Gelierzeiten. Dies gilt insbesondere bei niedrigen Temperaturen. Weiterhin zeigen erfindungsgemässe Zusammensetzungen bei Härtung unter gleichen Bedingungen einen besseren Härtungsgrad. Beschichtungen aus erfindungsgemässen Zusammensetzungen zeigen nach der Härtung bessere Haftfähigkeit als Mischungen des Standes der Technik. Dies ist z. B. von besonderem Vorteil für die Anwendung als Klebstoff.

In der Bedeutung "Alkyl" stellen R₁ bis R₉ in Formel 1 bevorzugt ein unverzweigtes oder verzweigtes Alkyl mit 1 bis 10 Kohlenstoffatomen dar, das gegebenenfalls auch einfach oder mehrfach substituiert sein kann, z. B. mit Hydroxyl-, Halogen- oder C₁-C₄-Alkyloxygruppen. Beispiele sind Methyl, Ethyl, Propyl, Isopropyl, n-Butyl, tert.-Butyl, Octyl und 2-Hydroxyethyl. Besonders bevorzugt als Alkyl ist C₁-C₄-Alkyl. In der Bedeutung "Aryl" stellen R₁ bis R₉ bevorzugt Arylgruppen mit 5 bis 10 Ringkohlenstoffatomen dar, die gegebenenfalls auch substituiert sein können, z. B. mit Halogen- oder C₁-C₄-Alkyl- oder C₁-C₄-Alkyloxygruppen, und auch Heteroatome, wie z.B. Stickstoff, Sauerstoff, Schwefel, enthalten können. Bevorzugt ist in diesem Fall Phenyl und substituiertes Phenyl. In der Bedeutung "Arylalkyl" stellen R₁ bis R₉ bevorzugt eine C₁-C₄-Alkylgruppe dar, die Arylsubstituenten, bevorzugt in der oben für "Aryl" angegebenen Bedeutung und bevorzugt in einer Anzahl von 1 oder 2 enthalten. Bevorzugter Arylalkylrest ist Benzyl. In der Bedeutung "Halogen" stellen R₁ bis R₉ bevorzugt Chlor oder Brom dar. Bedeuten R₂ zusammen mit R₃ oder R₅ zusammen mit R₆ oder R₈ zusammen mit R₉ einen an dieser Stelle annellierten Benzolring, so handelt es sich bei dem Imidazolderivat um Benzimidazol. Auch dies kann gegebenenfalls noch Substituenten aufweisen, z. B. eine oder mehrere Halogen- oder C₁-C₄-Alkyl- oder C₁-C₄-Alkyloxygruppen.

Erfindungsgemäss ganz besonders bevorzugt sind Zusammensetzungen auf Basis von Verbindungen der Formel 1, worin die Reste R₁ bis R₉ jeweils unabhängig voneinander Wasserstoff, Alkyl, insbesondere C₁-C₄-Alkyl, oder Aryl, insbesondere Phenyl, bedeuten.

Die Verbindungen der Formel 1 sind teilweise schon bekannt. Sie können z. B. gemäss oder analog dem Verfahren aus der US-A-3,308,122 hergestellt werden, indem man Cyanurchlorid in einem inerten Lösungsmittel, wie Tetrahydrofuran, Diethylether, Toluol, Xylol oder Cyclohexan, und in Anwesenheit eines Protonenakzeptors, z. B. einer nicht nukleophilen organischen Base wie Triethylamin, mit einer etwa stöchiometrischen Menge eines in gewünschter Weise substituierten Imidazol- oder Benzimidazolderivats umsetzt. Auch die zur Umsetzung benötigten Imidazol- oder Benzimidazolderivate selbst sind als Protonenakzeptor geeignet, wobei sie selbstverständlich insgesamt im Überschuss eingesetzt werden müssen. Das Ausgangsimidazol kann hierbei leicht zurückgewonnen werden; indem man die Imidazolidiniumlösung zunächst mit starker Base neutralisiert und danach mit einem geeigneten Lösungsmittel, z. B. eines der oben angeführten, extrahiert.

Für die Erfindung geeignete substituierte Imidazole sind in üblicher Weise, z. B. mit Hilfe der Radziszewski Reaktion erhältlich, gemäss der folgenden Reaktionsgleichung (für das Beispiel des Imidazolrestes aus Formel 1 mit den Resten R₁, R₂ und R₃): 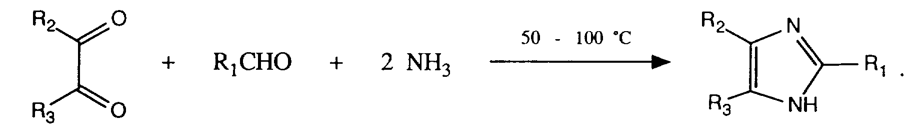 Viele Imidazolderivate sind auch kommerziell verfügbar. Wegen ihrer weiten Verbreitung und guten Verfügbarkeit sind Imidazol, 2-Methylimidazol, 4(5)-Methylimidazol, 2-Ethylimidazol, 2-Isopropylimidazol, 2-Ethyl-4(5)-methylimidazol, 2-Phenylimidazol und 2-Benzylimidazol besonders bevorzugt.

Weiterhin sind Zusammensetzungen auf Basis von Verbindungen der Formel 1 bevorzugt, worin die Reste R₁, R₄ und R₇ sowie die Reste R₂, R₅ und R₈ und die Reste R₃, R₆ und R₉ identische Bedeutung haben. Tris(benzimidazol-1-yl)triazin ist wegen seiner relativ schlechten Reaktivität ausgeschlossen. Diese Verbindungen sind in aller Regel Feststoffe mit Schmelzpunkten über 100 °C und besonders schwer löslich. Dies erleichtert nicht nur ihre Herstellung der Verbindungen sondern verbessert auch die Lagerstabilität der entsprechenden Zusammensetzungen.

Alle gebräuchlichen Di- und Polyepoxide sowie Epoxidharzpräpolymere sind als Epoxidharze für die Erfindung geeignet. Die Di- und Polyepoxide können aliphatische, cycloaliphatische oder aromatische Verbindungen sein. Beispiele für solche Verbindungen sind die Glycidylether und β-Methylglycidylether aliphatischer oder cycloaliphatischer Diole oder Polyole, zum Beispiel solche des Ethylenglykols, Propan-1,2-diols, Propan-1,3-diols, Butan-1,4-diols, Diethylenglykols, Polyethylenglykols, Polypropylenglykols, Glycerins, Trimethylolpropans oder 1,4-Dimethylolcyclohexans oder des 2,2-Bis-(4-hydroxycyclohexyl)-propans, die Glycidylether von Di- und Polyphenolen, beispielsweise Resorcin, 4,4'-Dihydroxydiphenylmethan, 4,4'-Dihydroxydiphenyl-2,2-propan, Novolake und 1,1,2,2-Tetrakis-(4-hydroxyphenyl)-ethan. Weitere Beispiele sind N-Glycidylverbindungen, z.B. die Diglycidylverbindungen des Ethylenharnstoffs, 1,3-Propylenharnstoffs oder 5-Dimethylhydantoins oder des 4,4'-Methylen-5,5'-tetramethyldihydantoins, oder solche wie Triglycidylisocyanurat.

Weitere Glycidylverbindungen mit technischer Bedeutung sind die Glycidylester von Carbonsäuren, insbesondere Di- und Polycarbonsäuren. Beispiele dafür sind die Glycidylester der Bernsteinsäure, Adipinsäure, Azelainsäure, Sebacinsäure, Phthalsäure, Terephthalsäure, Tetra- und Hexahydrophthalsäure, Isophthalsäure oder Trimellitsäure, oder von dimerisierten Fettsäuren.

Beispiele für von Glycidylverbindungen verschiedene Polyepoxide sind die Diepoxide des Vinylcyclohexens und Dicyclopentadiens, 3-(3',4'-Epoxicyclohexyl)-8,9-epoxy-2,4-dioxaspiro[5.5]undecan, der 3',4'-Epoxicyclohexylmethylester der 3,4-Epoxicyclohexancarbonsäure, Butadiendiepoxid oder Isoprendiepoxid, epoxidierte Linolsäurederivate oder epoxidiertes Polybutadien.

Bevorzugte Epoxidharze sind gegebenenfalls vorverlängerte Diglycidylether zweiwertiger Phenole oder zweiwertiger aliphatischer Alkohole mit 2 bis 4 Kohlenstoffatomen, insbesondere die gegebenenfalls vorverlängerten Diglycidylether des 2,2-Bis-(4-hydroxyphenyl)-propans und Bis-(4-hydroxyphenyl)-methans oder ein Gemisch aus diesen Epoxidharzen.

Obwohl Zusammensetzungen auf Basis einer Harzkomponente, die nur aus Epoxidharzen besteht, eine wichtige Ausführungsform der Erfindung darstellen, können neben den Epoxidharzen auch noch Polyisocyanate in den härtbaren Zusammensetzungen enthalten sein.

Als Polyisocyanate sind hierbei alle zur Vernetzung fähigen Polyisocyanate geeignet, z.B. Hexamethylendiisocyanat (HDI), Trimethylhexamethylendiisocyanat (TMDI), Cyclohexandiisocyanat (CHDI), Isophorondiisocyanat (3,5,5-Trimethyl-1-isocyanato-3-iscyanatomethylcyclohexan; IPDI), Methylendicyclohexylisocyanat (HMDI), p-Phenylendiisocyanat (PPDI), Diisocyanatotoluol (TDI), z. B. 2,4-Diisocyanatotoluol, 2,6 Diisocyanatotoluol und technische Gemische von beiden Isomeren, Naphthylendiisocyanat (NDI), insbesondere 1,5-Naphthylendiisocyanat, Dianisidindiisocyanat (DADI), Methylendiphenyldiisocyanat (MDI), insbesondere das 4,4'-Isomere, aber auch technische Gemische verschiedener Isomere, beispielsweise der 4,4'- und 2,4'-Isomeren, oder Polymethylenpolyphenylisocyanate (PAPI). Ebenfalls gut geeignet sind auch Polyisocyanate, die durch Umsetzung von Polyisocyanaten mit sich selbst über Isocyanatgruppen erhältlich sind, wie Uretdione oder Carbodiimidverbindungen, die durch Reaktion zweier Isocyanatgruppen entstehen, oder wie Isocyanurat- oder Biuretverbindungen, die durch Reaktion dreier Isocyanatgruppen entstehen. Für die Erfindung geeignet sind auch Polyisocyanatpräpolymere, die durchschnittlich mehr als eine Isocyanatgruppe pro Molekül aufweisen und durch Vorreaktion eines molaren Überschusses beispielsweise eines der oben genannten Polyisocyanate mit einem organischen Material erhalten werden, das mindestens zwei aktive Wasserstoffatome pro Molekül aufweist, z. B. in Form von Hydroxylgruppen wie bei Polyalkylenglykolen. Isocyanate wie die genannten sind allgemein verfügbar und in grosser Vielfalt kommerziell erhältlich.

Bevorzugt werden als Polyisocyanate Methylendiphenyldiisocyanat (MDI), Isophorondiisocyanat (IPDI), Hexamethylendiisocyanat, Diisocyanatotoluol (TDI), z. B. 2,4-Diisocyanatotoluol, 2,6 Diisocyanatotoluol und technische Gemische von beiden Isomeren, und Präpolymere der genannten Isocyanate mit Polyalkoholen sowie Umsetzungsprodukte, die durch Umsetzung der Polyisocyanate mit sich selbst über Isocyanatgruppen erhältlich sind.

Die freien Isocyanatgruppen der Polyisocyanate können auch in üblicher Weise blockiert sein, z. B. mit Phenolen, Oximen, Pyrazolen oder Indazolen. Pyrazolblockierte Polyisocyanate sind bevorzugt, weil sie bereits bei relativ niedrigen Temperaturen, ab etwa 80°C, wieder Isocyanat freisetzen und zu härten beginnen.

Die Herstellung pyrazolblockierter Polyisocyanate ist z. B. in der US-A-4,976,837 oder in der EP-A-0 500 495 beschrieben. Sie kann durch quantitative Umsetzung geeigneter Pyrazole mit den Polyisocyanaten unter Inertgas erfolgen. Vorzugsweise arbeitet man dabei bei erhöhter Temperatur und in einem geeigneten inerten Lösungsmittel (z. B. Toluol), gegebenenfalls in Gegenwart eines Katalysators (z. B. Dibutylzinndilaurat). Infolge der exothermen Reaktion beider Verbindungen kann eine Kühlung erforderlich sein. Die zur Blockierung verwendeten Pyrazole haben bevorzugt die Formel 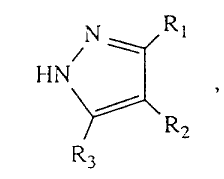 wobei R₁, R₂ und R₃ unabhängig voneinander Wasserstoff, Hydroxyl, Alkyl, insbesondere mit einem bis zu fünf Kohlenstoffatomen, Alkyloxy, insbesondere mit einem bis zu fünf Kohlenstoffatomen, Alkylthio, insbesondere mit einem bis zu fünf Kohlenstoffatomen, wobei wiederum Methyl und Methoxy besonders bevorzugt sind; Aryl mit bis zu zehn Ringkohlenstoffatomen, insbesondere Phenyl, wobei die Arylreste gegebenfalls ihrerseits Substituenten haben können, z. B. C₁-C₄-Alkyl- oder C₁-C₄-Alkyloxysubstituenten; oder Arylalkyl, insbesondere Aryl-(C₁-C₄)Alkyl, ganz besonders Arylmethyl, insbesondere Benzyl bedeuten. Pyrazole dieser Art sind im Handel oder können gemäss üblicher Methoden, z. B. durch Umsetzung passend gewählter i,i+2-Diketone, z. B. eines 1,3-Diketons, mit Hydrazin mit oder ohne Lösungsmittel (z. B. Toluol) erhalten werden.

Werden Gemische aus Epoxidharzen und Polyisocyanaten eingesetzt, so macht das Epoxidharz bevorzugt mindestens 50, noch besser mindestens 70 Gewichtsprozent der gesamten Harzkomponente aus.

Die Verbindungen der Formel 1 können den einzigen Härter der erfindungsgemässen Zusammensetzungen darstellen. In diesem Fall werden sie z. B. in einer Menge von 1 bis 10 Gewichtsprozent, bezogen auf das Epoxidharz beziehungsweise auf das Gemisch aus Epoxidharzen und Polyisocyanaten, angewandt.

Sie können aber auch in Gegenwart von anderen üblichen Härtern für Epoxidharze eingesetzt werden, wie z. B. von Dicyandiamid oder Aminhärtem, wie Imidazol. In diesem Fall sind geringere Mengen der Triazinverbindung ausreichend, und sie wird bevorzugt in einer Menge von 0,1 bis 5 Gewichtsprozent, ebenfalls bezogen auf das Epoxidharz beziehungsweise auf das Gemisch aus Epoxidharzen und Polyisocyanaten, angewandt.

Eine bevorzugte Ausführungsform der erfindungsgemässen Zusammensetzungen enthält Dicyandiamid als weitere Härterkomponente, z. B. in einer Menge von 5 bis 10 Gewichtsprozent, bezogen auf das Harz. Zusammensetzungen dieser Art haben zwar gute Klebefähigkeit und Lagerstabilität, sind aber nicht so reaktionsträge wie die entsprechenden Zusammensetzungen auf Basis von Dicyandiamid als einzigem Härter. Die Verbindungen der Formel 1, die hierbei bevorzugt in einer Menge von 0,5 bis 2,5 Gewichtsprozent, bezogen auf das Epoxidharz, vorhanden sind, wirken als Reaktionsbeschleuniger.

Neben den genannten Bestandteilen können die erfindungsgemässen Zusammensetzungen noch weitere übliche Bestandteile warmhärtender Zusammensetzungen in den üblichen Mengen enthalten, wie z. B. Viskositätsregler, Streckmittel, Füllstoffe, Verstärkungsmittel, Metallpartikel, Pigmente, Farbstoffe, organische Lösungsmittel, Weichmacher, Haftvermittler, Fungizide, Antioxidantien, Verlaufsmittel, Verdünner, z. B. Reaktivverdünner und dergleichen mehr.

Die erfindungsgemässen härtbaren Gemische lassen sich ganz allgemein zur Herstellung von gehärteten Produkten einsetzen, und können in einer dem jeweils speziellen Anwendungsgebiet angepassten Formulierung beispielsweise als Beschichtungsmassen, Lacke, Pressmassen, Tauchharze, Giessharze, Imprägnierharze, Laminierharze, 1- oder 2- Komponenten-Klebstoffe oder Matrixharze eingesetzt werden.
Sie eignen sich z. B. zur Herstellung von Prepregs, Verbund- oder Compositematerialien, zur Herstellung von Formkörpern jeglicher Art oder zum Umhüllen von elektrischen oder elektronischen Bauteilen. Ein besonderes Anwendungsgebiet für die Zusammensetzungen auf Basis von Mischungen aus Epoxidharzen und Polyisocyanaten ist die Herstellung gehärteter Produkte mit einreagierten Modifizierungsmitteln, wie Weichmachern oder Flexibilisatoren, wobei z. B. die Polyisocyanatkomponente das Modifizierungsmittel für die Epoxidharzkomponente darstellt (Interpenetrating Polymer Networks).

Besonders bevorzugt sind Anwendungen, bei denen es auf die Erreichung einer hohen Haftfähigkeit von gehärtetem erfindungsgemässen Material ankommt, d. h. die Verwendung der erfindungsgemässen Zusammensetzungen als Klebstoff, Beschichtungsmittel oder zur Herstellung von Prepregs. Für diese Anwendungen sind insbesondere Zusammensetzungen mit Verbindungen der Formel 1 geeignet, bei denen R₁ bis R₉ Wasserstoff, Alkyl- oder Arylreste darstellen.

Die erfindungsgemässen Gemische können bei relativ niedrigen Temperaturen rasch ausgehärtet werden. Im allgemeinen wendet man zur Aushärtung Temperaturen im Bereich von 20 bis 200°C, vorzugsweise von 60 bis 180°C, insbesondere 80 bis 120°C, an. Die Härtung kann dabei durch Zufuhr von Wärme in jeglicher Form erfolgen. Sie kann beispielsweise auch mit Hilfe von Mikrowellen oder durch Induktionserwärmung durchgeführt werden, wobei für letzteres die Zusammensetzungen selbstverständlich elektrisch leitende Partikel, z. B. Metallpartikel, enthalten müssen.

Ein besonderer Vorteil der erfindungsgemässen Zusammensetzungen liegt jedoch darin, dass schon bei besonders niedrigen Temperaturen im Bereich unter 120°C, insbesondere unter 110°C, mit für die Praxis ausreichender Geschwindigkeit ausgehärtet werden kann.

Die Gelierzeiten der Zusammensetzungen liegen nämlich im allgemeinen auch bei einer Temperatur von 100 - 120 °C unter 30 Minuten. Die Erfindung betrifft daher auch ein Verfahren zur thermischen Härtung von Epoxidharzen oder Gemischen von Epoxidharzen und Polyisocyanaten, bei dem eine Verbindung der Formel 1 entweder als einziger Härter oder in Verbindung mit Dicyandiamid als zweitem Härter eingesetzt wird und die Härtung bei einer Temperatur unter 120 °C durchgeführt wird. Die Aushärtung erfolgt in der Regel unter gleichzeitiger Formgebung zu Formkörpern, Imprägnierungen, Beschichtungen oder Verklebungen.

Die aus den erfindungsgemässen Gemischen durch thermische Härtung erhaltenen Produkte zeichnen sich vor allem durch einen hohen T_{G}-Wert und eine hohe Temperaturbeständigkeit aus. Ein weiterer Gegenstand der Erfindung sind somit auch die durch thermische Härtung der erfindungsgemässen Gemische erhaltenen Produkte.

Die folgenden Beispiele sollen die Erfindung weiter erläutern.

### Beispiel 1:

Im 3-Liter-Rundkolben mit Rückflusskühler, Stickstoffanschluss, Tropftrichter, Thermometer und mechanischer Rührung werden 5,28 Mol Imidazol (10-prozentiger Überschuss) in 1,2 Litern trockenem Tetrahydrofuran vorgelegt. Man tropft langsam und unter gutem Rühren eine Lösung von 0,8 Mol Cyanurchlorid in 0,8 Litern Tetrahydrofuran zu. Bei der exothermen Reaktion bildet sich eine Suspension, die 3 Stunden lang unter Rückfluss gekocht wird, danach auf Raumtemperatur abgekühlt und filtiert wird. Der Filtrationsrückstand wird mit kaltem Wasser gewaschen. Das Filtrat wird unter gutem Rühren in 4 Liter kaltes Wasser eingetropft, danach filtriert und der Filterrückstand ebenfalls mit kaltem Wasser gewaschen. Die vereinigten Rückstände werden im Hochvakuum bei 60 °C bis zur Gewichtskonstanz getrocknet. Man erhält reines Tris(imidazol-1-yl)triazin vom Schmelzpunkt 260 °C in 93-prozentiger Ausbeute.

| | | | | |
|---|---|---|---|---|
| Elementaranalyse: | ber. | C 51,61% | H 3,25% | N 45,14% |
| | gef. | C 51,72% | H 3,32% | N 45,16% |

¹H-NMR(δ[ppm]in CDCl₃): 8,7(s); 7,9(t, J=1,4Hz); 7,3(t, J=1,4Hz)

Die wässrigen Lösungen enthalten praktisch reines Imidazoliumhydrochlorid, das durch Eindampfen isoliert werden kann.

### Beispiel 2:

Im 1-Liter-Rundkolben mit Rückflusskühler, Stickstoffanschluss, Tropftrichter, Thermometer und mechanischer Rührung werden 0,9 Mol Imidazol und 0,945 Mol Triethylamin (5% Überschuss) in 0, 4 Litern trockenem Tetrahydrofuran vorgelegt. Man tropft langsam und unter gutem Rühren eine Lösung von 0,3 Mol Cyanurchlorid in 0,4 Litern Tetrahydrofuran zu. Bei der exothermen Reaktion bildet sich eine Suspension, die 3 Stunden lang unter Rückfluss gekocht wird, danach auf Raumtemperatur abgekühlt und filtiert wird. Der Filtrationsrückstand wird mit kaltem Wasser gewaschen. Das Filtrat wird unter starkem Rühren in 1,2 Liter kaltes Wasser eingetropft, danach filtriert und der Filterrückstand ebenfalls mit kaltem Wasser gewaschen. Die vereinigten Rückstände werden im Hochvakuum bei 60 °C bis zur Gewichtskonstanz getrocknet. Man erhält Tris(imidazol-1-yl)triazin, dessen Reinheit dem gemäss Beispiel 1 erhältlichen Produkt entspricht, in 89-prozentiger Ausbeute.

### Beispiel 3:

Es wird gemäss Beispiel 1 gearbeitet, nur dass 2-Methylimidazol anstatt Imidazol eingesetzt wird. Tris(2-methylimidazol-1-yl)triazin wird in 78-prozentiger Ausbeute erhalten. Beim Arbeiten gemäss der Vorschrift aus Beipiel 2 fällt das gleiche Produkt in 75-prozentiger Ausbeute an.

Schmelzpunkt: 266 °C.

| | | | | |
|---|---|---|---|---|
| Elementaranalyse: | ber. | C 56,07% | H 4,71% | N 39.23% |
| | gef. | C 55,48% | H 4,70% | N 39,34% |

¹H-NMR(δ[ppm]in CDCl₃): 7,88(d, J=1,8Hz); 7,06(d, J=1,8Hz); 2,95(s).

### Beispiel 4:

Es wird gemäss Beispiel 1 gearbeitet, nur dass 2-Ethyl-4-methylimidazol anstatt Imidazol eingesetzt wird. Es wird Tris(2-ethyl-4-methylimidazol-1-yl)triazin erhalten. Beim Arbeiten gemäss der Vorschrift aus Beipiel 2 fällt das Produkt in ähnlicher Ausbeute an.

Schmelzpunkt: 187 °C.

| | | | | |
|---|---|---|---|---|
| Elementaranalyse: | ber. | C 62,20% | H 6,71% | N 31,09% |
| | gef. | C 62,14% | H 6,79% | N 31,35% |

¹H-NMR(δ[ppm]in CDCl₃): 7,51(q, J=1,1Hz); 3,31(q, J=7,4Hz); 2,27(d, J=1,1Hz); 1,46(t, J=7,4Hz).

### Beispiel 5:

Es wird gemäss Beispiel 2 gearbeitet, nur dass 2-Phenylimidazol anstatt Imidazol eingesetzt wird. Tris(2-phenylimidazol-1-yl)triazin wird in 59-prozentiger Ausbeute erhalten.

### Beispiel 6:

Es wird gemäss Beispiel 1 gearbeitet, nur dass 2-Phenylimidazol anstatt Imidazol ein gesetzt wird. Tris(2-phenylimidazol-1-yl)triazin wird diesmal in 84-prozentiger Ausbeute erhalten.

Schmelzpunkt: 193 °C.

| | | | | |
|---|---|---|---|---|
| Elementaranalyse: | ber. | C 70,99% | H 4,17% | N 24,84% |
| | gef. | C 70,24% | H 4,60% | N 24,22% |

¹H-NMR(δ[ppm]in DMSO-d₆): 7,59-7,47(m, 15H); 7,06(d, J=1,4Hz, 3H); 6,64(d, J=1,4Hz, 3H).

### Beispiele 7 - 14:

Der feinpulverisierte Härter aus dem in Tabelle 1 jeweils angegebenen Beispiel wird mit einem Epoxidharz auf Basis von Bisphenol A (Epoxidäquivalent 185 - 190 g/Äqu; Viskosität(bei 25 °C nach DIN 53015): 10000 - 12000 mPas; Araldit^{®}GY-250) und gegebenenfalls dem in Tabelle 1 angegebenen Co-Härter vermischt und zweimal über den 3-Walzenstuhl gelassen. Die Mischungen haben die in Tabelle 1 angegebenen Eigenschaften.

### Beispiele 15 - 19:

Es wird analog zu den Beispielen 7 bis 14 gearbeitet, jedoch werden erfindungsgemässe Hybridsysteme auf Basis von Epoxidharzen und der folgenden Polyisocyanate eingesetzt, bei denen jeweils alle freien Isocyanatgruppen blockiert sind.
- PIC 1 =: Präpolymer, erhalten durch Umsetzung von 22,58 Gew.-% Methylendiphenyldiisocyanat (Isonate^{®}M 125 [DOW]), 67,72 Gew.-% eines linearen Polyetherpolyols auf Basis von Polypropylenglycol (Desmophen^{®}1900 U [BAYER]) und blockiert mit 7,73 Gew.-% 3,5-Dimethylpyrazol und 1,52 Gew.-% Imidazol in Gegenwart von 0,45 Gew.-% Dibutylzinndilaurat (10-prozentig in Xylol).
- PIC 2 =: Präpolymer, erhalten durch Umsetzung von 30,9 Gew.-% Methylendiphenyldiisocyanat (isonate^{®}M 125 [DOW]), 47,0 Gew.-% eines linearen Polyetherpolyols auf Basis von Polypropylenglycol (Desmophen^{®}1900 U [BAYER]) und blockiert mit 18,0 Gew.-% 3,5-Dimethylpyrazol ( 4,1 Gew.-% Dibutylzinndilaurat; 10-prozentig in Xylol).
- PIC 3 =: Präpolymer, erhalten durch Umsetzung von 9,92 Gew.-% Toluenediisocyanat, 86,47 Gew.-% eines Polytetrahydrofurans mit einem Molekulargewicht von ca. 2000 und blockiert mit 2,65 Gew.-% 3,5-Dimethylpyrazol ( 0,96 Gew.-% Dibutylzinndilaurat).

Die Mischungen haben die in Tabelle 2 angegebenen Eigenschaften.

In Tabelle 2 bedeuten weiterhin:

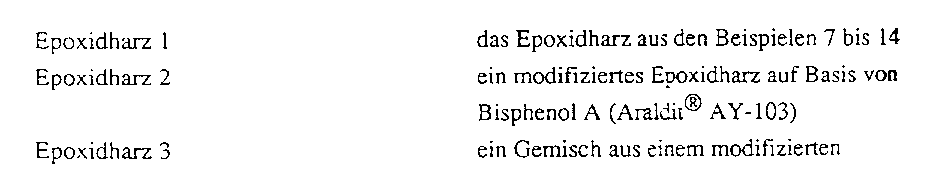

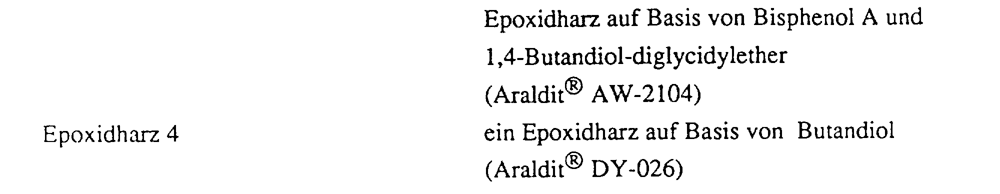

In den nachfolgenden Tabellen 1 und 2 beziehen sich die Werte für die Zugscherkraft auf eine vollständige Aushärtung (60-minütige Härtung bei 160 °C). Als Mass für die Stabilität ist in den Tabellen der Zeitraum angegeben, der jeweils verstreicht, bis sich die Viskosität bei der angegebenen Temperatur verdoppelt hat.

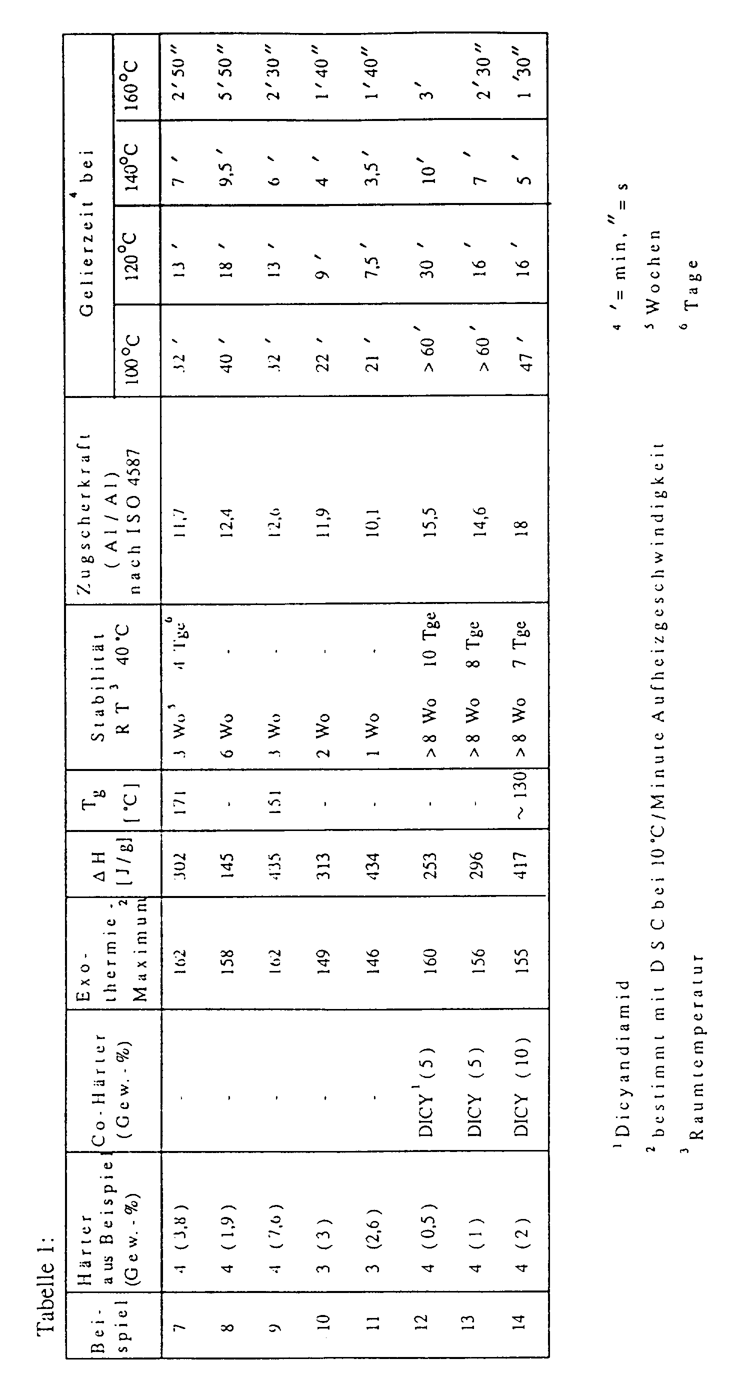

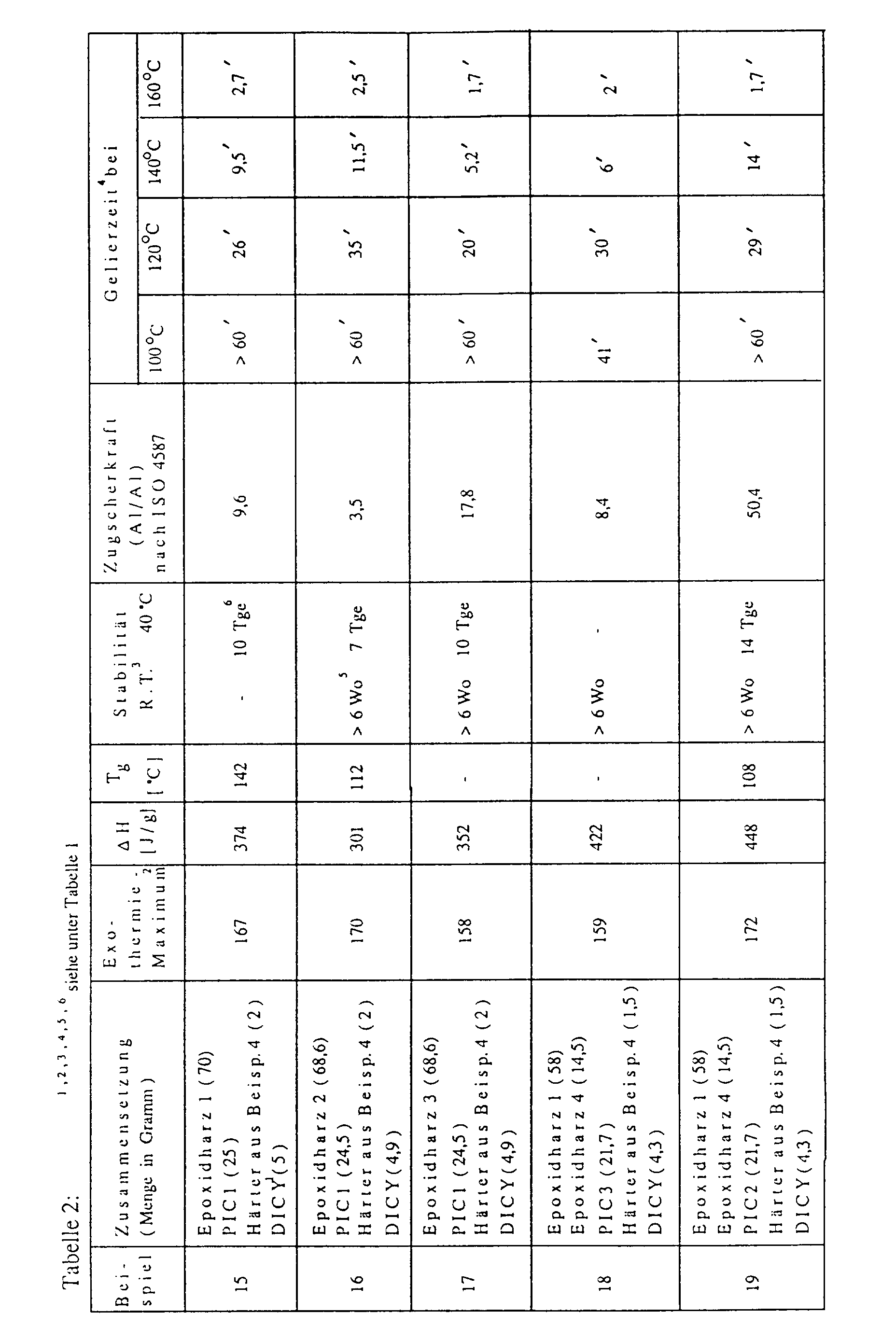

## Patentansprüche

1. Härtbare Zusammensetzung auf Basis von Epoxidharzen oder Gemischen von Epoxidharzen und Polyisocyanaten, die mindestens eine Verbindung der Formel 1 enthalten: 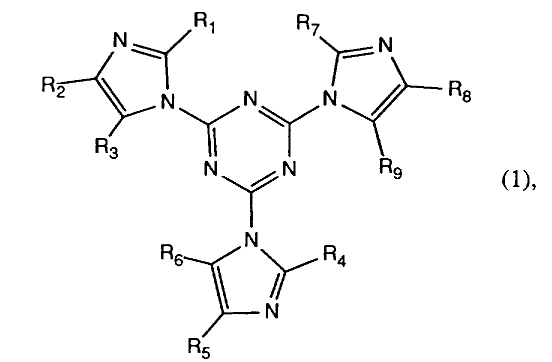 worin R₁, R₂, R₃, R₄, R₅, R₆, R₇, R₈ und R₉ jeweils unabhängig voneinander Wasserstoff, Halogen, unsubstituiertes oder substituiertes Alkyl oder Aryl oder Aryl-(C₁-C₄)alkyl, wobei der Arylrest unsubstituiert oder substituiert sein kann, bedeuten und R₂ zusammen mit R₃ sowie R₅ zusammen mit R₆ und R₈ zusammen mit R₉ ausserdem auch jeweils einen an dieser Stelle annellierten Benzolring darstellen können, wobei diese Benzimidazolringe unsubstituiert oder substituiert sein können und Tris(benzimidazol-1-yl)triazin ausgeschlossen ist.

2. Zusammensetzung nach Anspruch 1 auf Basis von Verbindungen der Formel 1, worin die Reste R₁ bis R₉ jeweils unabhängig voneinander Wasserstoff, Alkyl, insbesondere C₁-C₄-Alkyl, oder Aryl, insbesondere Phenyl, bedeuten.

3. Zusammensetzung nach einem der Ansprüche 1 oder 2 auf Basis von Verbindungen der Formel 1, worin die Reste R₁, R₄ und R₇ sowie die Reste R₂, R₅ und R₈ und die Reste R₃, R₆ und R₉ jeweils identische Bedeutung haben.

4. Zusammensetzung nach mindestens einem der Ansprüche 1 bis 3 auf Basis einer Harzkomponente, die nur aus Epoxidharzen besteht.

5. Zusammensetzung nach mindestens einem der Ansprüche 1 bis 3, die pyrazolblockierte Polyisocyanate enthält.

6. Zusammensetzung nach mindestens einem der Ansprüche 1 bis 5, die die Verbindung der Formel 1 in einer Menge von 1 bis 10 Gewichtsprozent enthält, wobei die Mengenangabe auf das Epoxidharz oder, wenn ein Polyisocyanat zugegen ist, auf das Gemisch aus Epoxidharz und Polyisocyanat bezogen ist.

7. Zusammensetzung nach mindestens einem der Ansprüche 1 bis 5, die einen weiteren Härter enthält und die Verbindung der Formel 1 in einer Menge von 0,1 bis 5 Gewichtsprozent, wobei die Mengenangabe auf das Epoxidharz oder, wenn ein Polyisocyanat zugegen ist, auf das Gemisch aus Epoxidharz und Polyisocyanat bezogen ist.

8. Zusammensetzung nach Anspruch 7, die Dicyandiamid als weiteren Härter enthält.

9. Verfahren zur thermischen Härtung von Epoxidharzen oder Gemischen von Epoxidharzen und Polyisocyanaten, bei dem mindestens eine Verbindung der Formel 1 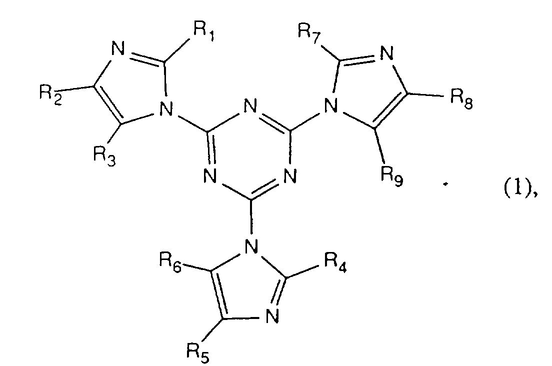 worin R₁, R₂, R₃, R₄, R₅, R₆, R₇, R₈ und R₉ jeweils unabhängig voneinander Wasserstoff, Halogen, unsubstituiertes oder substituiertes Alkyl oder Aryl oder Aryl-(C₁-C₄)alkyl, wobei der Arylrest unsubstituiert oder substituiert sein kann, bedeuten und R₂ zusammen mit R₃ sowie R₅ zusammen mit R₆ und R₈ zusammen mit R₉ ausserdem auch jeweils einen an dieser Stelle annellierten Benzolring darstellen können, wobei diese Benzimidazolringe unsubstituiert oder substituiert sein können und Tris(benzimidazol-1-yl)triazin ausgeschlossen ist, entweder als einziger Härter oder in Verbindung mit Dicyandiamid als zweitem Härter eingesetzt wird und die Härtung bei einer Temperatur unter 120 °C durchgeführt wird.

10. Gehärtetes Produkt, erhältlich durch thermische Härtung einer Zusammensetzung nach mindestens einem der Ansprüche 1 bis 8.

## Claims

1. A curable composition based on epoxy resins or on mixtures of epoxy resins and polyisocyanates, containing at least one compound of formula 1: 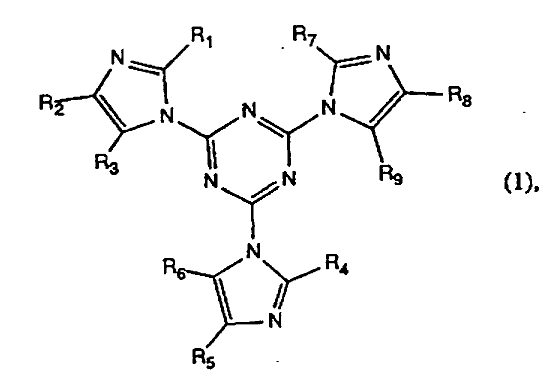 wherein R₁, R₂, R₃, R₄, R₅, R₆, R₇, R₈ and R₉ are each independently of one another hydrogen, halogen, unsubstituted or substituted alkyl or aryl or aryl-C₁-C₄alkyl, in which the aryl radical can be unsubstituted or substituted, and R₂ together with R₃ and R₅ together with R₆ and R₈ together with R₉ may also each represent a benzene ring which is fused at this point, it being possible for these benzimidazole rings to be unsubstituted or substituted, and excluding tris(benzimadazol-1-yl)triazine.

2. A composition according to claim 1, based on compounds of formula 1 wherein the radicals R₁ to R₉ are each independently of one another hydrogen, alkyl, especially C₁-C₄alkyl, or aryl, especially phenyl.

3. A composition according to either of claims 1 and 2, based on compounds of formula 1 wherein the radicals R₁, R₄, and R₇ and the radicals R₂, R₅ and R₈ and the radicals R₃, R₆ and R₉ are each identical.

4. A composition according to at least one of claims 1 to 3, based on a resin component consisting only of epoxy resins.

5. A composition according to at least one of claims 1 to 3, which contains pyrazole-blocked polyisocyanates.

6. A composition according to at least one of claims 1 to 5, which contains the compound of formula 1 in an amount of 1 to 10 per cent by weight, based on the epoxy resin or, if a polyisocyanate is present, on the mixture of epoxy resin and polyisocyanate.

7. A composition according to at least one of claims 1 to 5, which contains an additional hardener and the compound of formula 1 in an amount of 0.1 to 5 per cent by weight, based on the epoxy resin or, if a polyisocyanate is present, on the mixture of epoxy resin and polyisocyanate.

8. A composition according to claim 7, which contains dicyandiamide as additional hardener.

9. A process for the thermal curing of epoxy resins or mixtures of epoxy resins and polyisocyanates, which comprises using at least one compound of formula 1 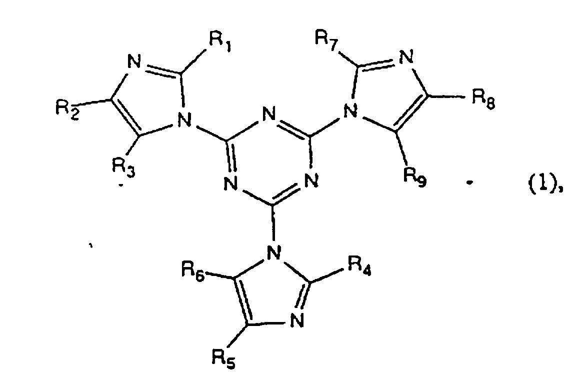 wherein R₁, R₂, R₃, R₄, R₅, R₆, R₇, R₈ and R₉ are each independently of one another hydrogen, halogen, unsubstituted or substituted alkyl or aryl or aryl-C₁-C₄alkyl, in which the aryl radical can be unsubstituted or substituted, and R₂ together with R₃ and R₅ together with R₆ and R₈ together with R₉ may also each represent a benzene ring which is fused at this point, it being possible for these benzimidazole rings to be unsubstituted or substituted, and excluding tris(benzimadazol-1-yl)triazine, either as sole hardener or in conjunction with dicyandiamide as second hardener, and carrying out the cure at a temperature below 120°C.

10. A cured product obtainable by thermally curing a composition according to at least one of claims 1 to 8.

## Revendications

1. Composition durcissable à base de résines époxy ou de mélanges de résines époxy et de polyisocyanates, qui contient au moins un composé de formule 1: 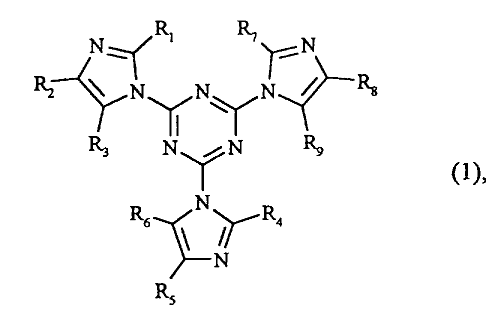 dans laquelle R₁, R₂, R₃, R₄, R₅, R₆, R₇, R₈ et R₉ représentent chacun, indépendamment les uns des autres, un atome d'hydrogène ou d'halogène ou un reste alkyle ou aryle non substitué ou substitué, ou aryl(alkyle en C₁-C₄) dont le reste aryle peut être non substitué ou substitué, et R₂ peut en outre former avec R₃, comme R₅ avec R₆ et R₈ avec R₉, un noyau benzène condensé en cette position, ces noyaux benzimidazole pouvant être non substitués ou substitués, et la tris(benzimidazole-1-yl)triazine étant exclue.

2. Composition selon la revendication 1, à base de composés de formule 1 dans lesquels les restes R₁ à R₉ représentent chacun, indépendamment les uns des autres, un atome d'hydrogène ou un reste alkyle, en particulier alkyle en C₁-C₄, ou aryle, en particulier phényle.

3. Composition selon l'une des revendications 1 ou 2, à base de composés de formule 1 dans lesquels les restes R₁, R₄ et R₇, ainsi que les restes R₂, R₅ et R₈ et les restes R₃, R₆ et R₉ ont à chaque fois la même signification.

4. Composition selon l'une au moins des revendications 1 à 3, à base d'un constituant résineux qui n'est composé que de résines époxy.

5. Composition selon l'une au moins des revendications 1 à 3, qui contient des polyisocyanates bloqués par des pyrazoles.

6. Composition selon l'une au moins des revendications 1 à 5, qui contient le composé de formule 1 en une quantité de 1 à 10 % en masse, cette quantité étant rapportée à la résine époxy ou, lorsqu'un polyisocyanate est présent, au mélange de la résine époxy et du polyisocyanate.

7. Composition selon l'une au moins des revendications 1 à 5, qui contient un autre durcisseur et le composé de formule 1 en une quantité de 0,1 à 5 % en masse, cette quantité étant rapportée à la résine époxy ou, lorsqu'un polyisocyanate est présent, au mélange de la résine époxy et du polyisocyanate.

8. Composition selon la revendication 7, qui contient du dicyandiamide comme autre durcisseur.

9. Procédé de durcissement thermique de résines époxy ou de mélanges de résines époxy et de polyisocyanates, selon lequel on utilise au moins un composé de formule 1 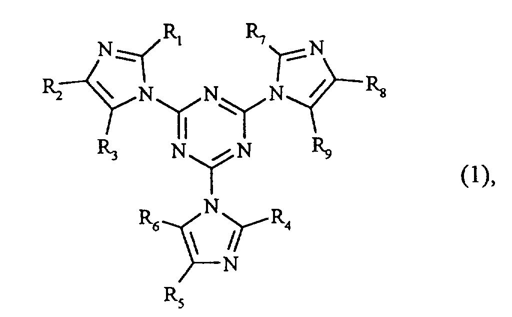 dans laquelle R₁, R₂, R₃, R₄, R₅, R₆, R₇, R₈ et R₉ représentent chacun, indépendamment les uns des autres, un atome d'hydrogène ou d'halogène ou un reste alkyle ou aryle non substitué ou substitué, ou aryl(alkyle en C₁-C₄) dont le reste aryle peut être non substitué ou substitué, et R₂ peut en outre former avec R₃, comme R₅ avec R₆ et R₈ avec R₉, un noyau benzène condensé en cette position, ces noyaux benzimidazole pouvant être non substitués ou substitués, et la tris(benzimidazole-1-yl)triazine étant exclue,
comme unique durcisseur ou en combinaison avec du dicyandiamide comme second durcisseur, et on effectue le durcissement à une température inférieure à 120°C.

10. Produit durci pouvant être obtenu par durcissement thermique d'une composition selon l'une au moins des revendications 1 à 8.
